(19) Europäisches Patentamt
European Patent Office
Office européen des brevets

(11) **EP 4 704 093 A1**

(12) **EUROPEAN PATENT APPLICATION**

(43) Date of publication:
**04.03.2026 Bulletin 2026/10**

(21) Application number: **25199108.9**

(22) Date of filing: **29.08.2025**

(51) International Patent Classification (IPC):
**G16C 10/00** (2019.01)   **G16C 20/30** (2019.01)
**G16C 20/70** (2019.01)

(52) Cooperative Patent Classification (CPC):
**G16C 10/00; G16C 20/30; G16C 20/70;**
**G16C 60/00**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **30.08.2024 US 202418821236**

(71) Applicant: **Robert Bosch GmbH**
**70442 Stuttgart (DE)**

(72) Inventors:
• **Kornbluth, Mordechai**
  **Brighton, MA, 02135 (US)**

• **Kitchaev, Daniil**
  **Newton, MA, 02458 (US)**
• **Molinari, Nicola**
  **Somerville, MA, 02143 (US)**
• **Gadelrab, Karim**
  **Wellesley, MA, 02481 (US)**
• **Tuffile, Charles**
  **Swansea, MA, 02777 (US)**

(74) Representative: **Isarpatent**
**Patent- und Rechtsanwälte**
**Barth Hassa Peckmann & Partner mbB**
**Friedrichstraße 31**
**80801 München (DE)**

(54) **MACHINE LEARNING AND MOLECULAR MECHANICS SIMULATION METHODS**

(57)    A machine learning interatomic potential (MLIP) method of determining a physical state of interaction between atoms from one or more physical properties of the atoms is disclosed. The method includes assigning a charge $q_i$ to a first subset of atoms via the MLIP dependent on a nonconstant field generated by a second subset of atoms having a charge $q_j$ to determine the physical state of interaction between the atoms. The method may include using the physical state of interaction between the atoms to control the chemical system.

FIG. 1

EP 4 704 093 A1

**Description**

TECHNICAL FIELD

The present disclosure relates to machine learning and molecular mechanics simulation methods.

BACKGROUND

Ab-initio calculations (e.g., density functional theory, otherwise referred to as DFT) have been used to obtain accurate estimations of interaction energies of a material at an atomistic scale. These ab-initio calculations are computationally expansive, thereby having a high magnitude of computational cost. Machine learned interatomic potentials (MLIPs) may be used to develop shorter-range models that can infer interaction energies with accuracy close to ab-initio calculations while reducing the computational costs by several orders of magnitude. MLIPs have shown promise as a compromise between accuracy and computational efficiency.

SUMMARY

A machine learning interatomic potential (MLIP) method of determining a physical state of interaction between atoms from one or more physical properties of the atoms is disclosed. The method includes assigning a charge $q_i$ to a first subset of atoms via the MLIP dependent on a nonconstant field generated by a second subset of atoms having a charge $q_j$ to determine the physical state of interaction between the atoms. The method may include using the physical state of interaction between the atoms to control the chemical system.

In another embodiment, a MLIP method of determining a physical state of interaction between atoms from one or more physical properties of the atoms is disclosed. The method includes assigning charges to a first subset of atoms in a molecular mechanics region via the MLIP to generate an electric field in a machine learning region to determine the physical state of interaction between the atoms.

BRIEF DESCRIPTION OF THE DRAWINGS

Figure 1 depicts a path of information from descriptors to output, including embedding and machine learning.

Figure 2 depicts a deep learning network that predicts forces using backpropagation.

Figure 3 is a flowchart depicting an example of a molecular dynamics algorithm using a backpropagation approach.

Figure 4A depicts a quantum mechanics/molecular mechanics (QM/MM) method including an MM region, a QM region, and an interface region therebetween.

Figure 4B depicts a mechanical embedding method where a QM solver does not account for charge outside of the QM region in the interface but the QM solver has visibility to bonds.

Figure 4C depicts an electrostatic embedding method where the QM solver has visibility to some charges in an interface outside of the QM region to repolarize atoms.

Figure 5 is a schematic view of a system geometry of an MLIP simulation according to one or more embodiments.

Figure 6A depicts a machine learning/molecular mechanics (ML/MM) simulation method including an ML region, an MM region, and an interface therebetween.

Figure 6B depicts a mechanical embedding method where the ML solver does not account for charge outside of the ML region in the interface but it can see bonds.

Figure 6C depicts an electronic embedding method where the ML solver sees some charges in the interface outside of the ML region to repolarize atoms.

Figure 7 depicts an information path for a ML/MM simulation method.

Figure 8 depicts a training method for training a machine learning interatomic potential (MLIP) according to one embodiment.

Figures 9 depicts a ML/MM simulation method including a ML region, a MM region, and an interface therebetween where the ML/MM simulation method may be used to calculate a total energy E from the equations shown in this figure.

Figure 10 depicts a schematic diagram of an in-space hybrid machine learning model.

Figure 11 depicts a schematic diagram of an in-time hybrid machine model.

Figure 12 depicts a computing platform for implementing the machine learning methods of one or more embodiments.

DETAILED DESCRIPTION

As required, detailed embodiments of the present invention are disclosed herein; however, it is to be understood that the disclosed embodiments are merely exemplary of the invention that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present invention.

The term "substantially" may be used herein to describe disclosed and/or claimed embodiments. The term "substantially" may modify a value or relative characteristic disclosed or claimed in the present disclosure. In such instances, "substantially" may signify that the value or relative characteristic it modifies is within $\pm$ 0%, 0.1%, 0.5%, 1%, 2%, 3%, 4%, 5% or 10% of the value or relative characteristic.

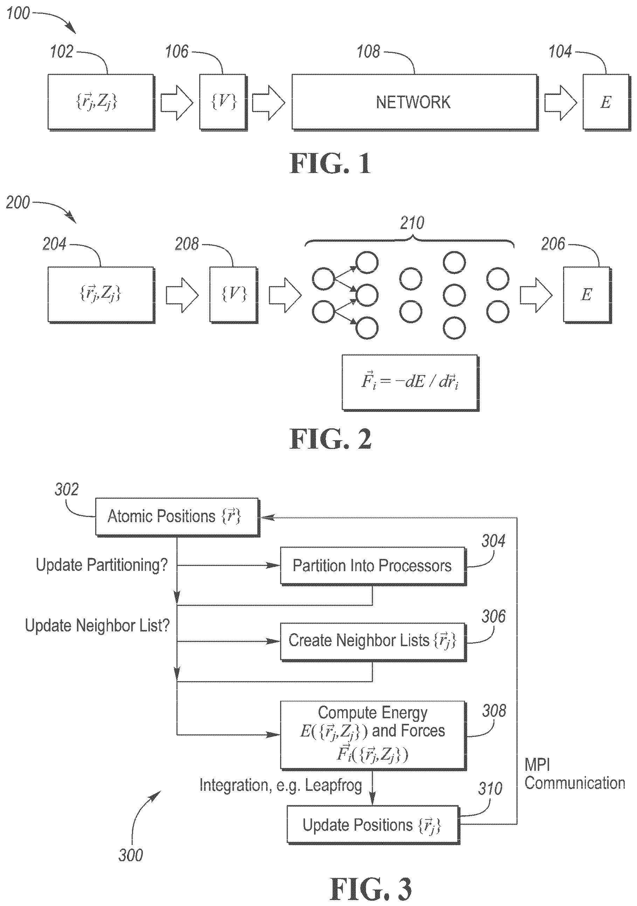

Machine learning interatomic potentials (MLIP) are techniques that use machine learning to map a set of atomic positions $\{\vec{r_i}\}$ and atomic species (e.g., atomic numbers and/or isotopes) $\{Z_i\}$ to a scalar energy E and/or forces $\{\vec{F_i}\}$. MLIPs may be used to determine a potential energy surface (PES) and/or compute forces. The scalar energy E and/or forces $\{\vec{F_i}\}$ may be used in computational simulations (e.g., reconstructing structures from experimental data, molecular dynamics, finding a ground state of a system, and/or identifying likely reactions and transition states). Figure 1 depicts path 100 of information from descriptors 102 to output 104, including embedding 106 and machine learning 108.

Two main classes of MLIP are Gaussian Processes (GP) and deep learning (DL) networks. A non-limiting example of a GP is FLARE, which has been developed by the Materials Intelligence Group (MIR) at Harvard University. A non-limiting example of a DL is Nequip, which is an open source code for building E(3)-equivariant interatomic potentials.

A DL network typically has several layers that are learned using backpropagation where the weights of the network are optimized to minimize a loss function. The loss function may be the function represented by equation (1) below.

$$L = \left| E_{pred} - E_{actual} \right|^2 \tag{1}$$

where $E_{actual}$ is training data extracted from a higher-fidelity method (e.g., density functional theory, otherwise referred to as DFT). The same backpropagation method(s) (e.g., PyTorch auto grad available in PyTorch, which is an open source machine learning (ML) framework based on the Python programming language and the Torch library) may be used to predict forces as the derivative of the total energy with respect to atomic positions. The DL may also be trained on forces using the function represented by equation (2) below.

$$L = \sum_i \left| \vec{F}_{i,pred} - \vec{F}_{i,actual} \right|^2 \tag{2}$$

Where $\vec{F}_{i,actual}$ are training data extracted from a higher-fidelity method such as DFT. The loss function may focus on a stress tensor. The loss function may also be a combination of two or more of the above loss functions. Figure 2 depicts deep learning network 200 that predicts forces using backpropagation. Deep learning network 200 depicts a path of information from atomic species and positions 204 to output 206, including descriptor embedding 208 and machine learning 210.

Figure 3 is flowchart 300 depicting an example of a molecular dynamics algorithm using a MLIP. Operation 302 of flowchart 300 enumerates the atomic positions $\{\vec{r_i}\}$ of a system (e.g., a molecule). Operation 304 partitions the atoms into processors to make it likely that adjacent atoms are on the same processor. Operation 304 reduces the amount of message passing interface (MPI) communication while carrying out the molecular dynamics algorithm. Partitioning may be used every N timesteps (e.g., N equals 1,000). Operation 306 creates a neighbor list of a set of all atoms j within a neighborhood of atom i (e.g., all atoms within a cutoff radius $r_c$). The neighbor list may be updated very M timesteps (e.g., M equals 100). Operation 308 computes the energy through any function that takes atomic positions, species, charges, and/or other properties, and outputs a total energy. Forces may be similarly computed (e.g., with a backprop from neural network energy). Operation 310 updates the positions $\{\vec{r_j}\}$ (e.g., using a leapfrog integration). An MPI communicates results between processors (e.g., updated positions and total energy).

One or more of the machine learning methods identified above that compute energy and/or forces may have one or more shortcomings. For example, for very large systems, the DL networks may not be scalable. Typically, the machine learning network takes a cutoff of neighbor atoms ($r_c$), where information in the network is passed only between atoms within the cutoff distance. However, common message-passing networks such as Nequip apply this cutoff at each level of the network, so that a network with N layers has an effective cutoff of $Nr_c$ and a number of effective neighboring atoms that scales as $(Nr_c)^3$. This scaling makes it impractical to partition the atoms in the system across different processors in a production run as well as computationally cubically expensive (e.g., the number of atoms increases with the cube of N). The cutoff-scaling issue may be partially solved by Allegro (which is another MLIP developed by MIR at Harvard University). Allegro partitions energy into a per-atom energy, $E_i$, represented by equation (3) below.

$$E_i = \sum_{j \in N_i} E_{ij}(N_i) \tag{3}$$

where $N_i$ is a set of all atoms in the neighborhood of i (e.g., within the cutoff) and no others, and $E_{ij}$ is an effective pairwise energy corresponding to two atoms i and j. Using this process, energy may be efficiently partitioned onto processors based on the atomic environment and neighbors.

As another shortcoming, these machine learning methods may not capture long-range effects such as electrostatics, especially in the purely local energy Allegro model. The energies and forces associated with these phenomena are strongly affected by longer range considerations than what can be captured with a cutoff radius $r_c$, so either the value of $r_c$ has to be increased enormously (with associated downsides, e.g., increasing instabilities, computation time, and memory requirements), or the long range effects have to be neglected, leading to a loss in the accuracy of the machine learning method.

Several proposals have attempted to resolve this shortcoming by having an auxiliary network learn electrostatic point charges from DFT, which may then be fed into a method for computing long-range electrostatic forces and energies such as the Ewald summation. Point charges may be fitted to DFT-based charges suing a Hirshfeld or Mulliken charge partitioning schemes, among others. Other proposals have derived effective charge values from other quantities such as fitting only to the total energy. A common drawback of current proposals is that the atomic charges may fluctuate often and considerably, and therefore, the entire potential energy surface may be extremely sensitive to the initial configuration of the simulation as well as small perturbations in the atomic positions over the course of the simulation.

Yet another shortcoming is discontinuities and/or roughness may not be effectively captured in the PES. Often there are segments of the PES that are smooth with respect to atomic position, and other segments that correspond to a transition (e.g., magnetic transition, bond breaking, and/or charge transfer) where there should be an abrupt change in the PES or its derivatives. The PES is still continuously differentiable to have a well-defined force.

Another challenge in molecular dynamics is assigning physical charges to atoms. The electrons themselves may be quantum-mechanical delocalized clouds, whereas in molecular dynamics the atoms have individual point sites and usually point charges. These may be implemented with several different schemes. For example, one approach is to fit the charges to some macroscopic behavior such as stress/strain relationships. A potential downside is that these relationships are not transferable between different materials, even for different phases of the same chemical composition. Another approach may include applying a delocalized charge to the atoms (e.g., a polarizable charge attached on a spring to the nucleus). However, this approach is computationally expensive and even harder to parametrize accurately.

Yet another approach is to extract the charge from a quantum mechanical calculation that treats the electrons explicitly. Within this approach, there are several types of "charge schemes" that are commonly used. The Born effective charge describes how much of a system's charge is bound to a particular atom's position (i.e., how much of the charge redistributes when the atom moves). This value may be calculated using density functional perturbation theory (DFPT) using the following equation (4):

$$Z_{ij}^* = -\frac{1}{e}\, \partial^2 E / \partial \varepsilon_i \partial u_j \tag{4}$$

The change in total energy is determined when an electric field $\varepsilon$ is applied in the direction i and atom j is moved. While this is quantitatively meaningful, it does not often correspond to the electrostatic forces caused by screened charges. The Mulliken charge may partition charges when the electron basis set is atom-centered. If the electron basis functions are centered at each atom, the total charge on atom j is the sum of all electron occupancies that are centered on that atom. The Lowdin charge may be used to be less dependent on the basis choice. Using the Mulliken charge often does not correspond to the electrostatic forces. The Bader charge may also be used. The Bader charge is partitioned based on the surfaces of minimum charge density. The Charge Equilibration via Interactive Partial Gradient (ChEIPG) scheme attempts to achieve a best fit to an electrostatic potential far from the molecule, which is often usable in molecular dynamics assignments. The Hirshfeld population analysis may be used to compare the electron density to a reference density to identify the excess charge due to the local environment. Other offshoots include the iterative Hirshfeld and CM5 variations.

Another proposal takes a different approach to dealing with charges. Instead of computing point charges, the proposal looks at the polarizability of the entire system computed by DFT. One key idea from this proposal is to apply a finite electric field $\varepsilon$ and run the DFT simulation, thereby computing the changes in energy and forces for different electric fields.

Another area of molecular dynamics is the quantum mechanics/molecular mechanics (QM/MM) framework, which attempts to address the problem of poorly-scaled highly-accurate models. In the QM/MM framework, there is a QM region that is treated with high accuracy models such as DFT and an MM region that does not have explicit electrons and is treated in an approximate way.

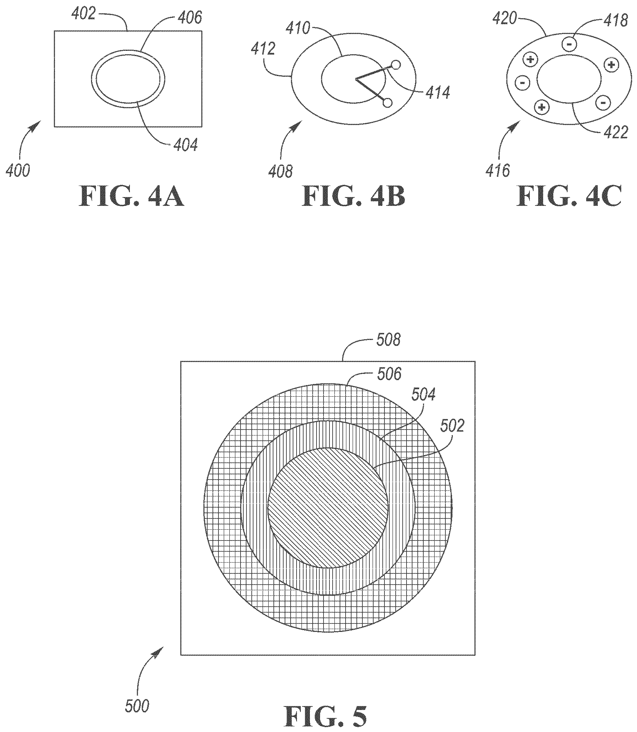

Figure 4A depicts QM/MM method 400 including MM region 402, QM region 404, and interface 406 therebetween. Figure 4B depicts mechanical embedding method 408 where the QM solver does not account for charge outside of QM region 410 in interface 412 but bonds 414 are visible. Figure 4C depicts electrostatic embedding method 416 where charges 418 in interface 420 are visible to the QM solver outside QM region 422 to repolarize atoms.

The total energy of the QM/MM framework may be described by equation (5).

$$E_{total} = E_{MM} + E_{QM} + E_{interface} \qquad (5)$$

The QM/MM methods may be divided into mechanical embedding and electrostatic embedding. In all schemes considered, the MM region assigns charges and bonds to each atom, and the QM region assigns atomic positions and solves for electron density. In mechanical embedding, the QM solver does not include any charge outside of the QM region. It typically has visibility to bonds in some form, leading to some variation in schemes to cap the bonds in a physical way (e.g., terminating all bonds with hydrogen). In electrostatic embedding, the QM solver sees some charges outside the QM region, which leads to the electron density in the QM region responding to the charges. This may be a combination of electrostatic density and assigned charges that are near the interface region. In some schemes, the charges passed to the QM solver are attenuated or scaled based on the positions or connectivity of the atoms.

Some recent proposals have suggested machine learning molecular mechanics (ML/MM) embedding, where there is a high-fidelity MLIP region and a low fidelity classical MD region. For instance, one proposal includes the electrostatic potential from MM atoms in the list of input features of the MLIP. The training data only includes the QM/MM training data and does not include any explicit electric field. This training data may limit transferability to other systems as well as the training data generation. Also, the output of the MLIP is only the total energy, not charges. Another proposal uses QM/MM with electrostatic embedding as the reference data. The atomic charges are extracted from those QM/MM data but are not affected by the field generated by MM atoms. Another proposal introduces FieldSchnet, which includes fields in the atomic descriptors. However, there is no training data or output on atomic point charges. Yet another proposal does ML/MM simulations configured to stop and query a machine learning model, thereby performing an in-time hybridization. The machine learning model interpolates between known force-field parameters and guesses the missing ones based on the updated bond topology. Yet another approach focuses on a bond breaking molecular dynamics machine learning (MDML) scheme. The machine learning model is pre-trained on already-known topologies, focuses on bond breaking geometries, does not automatically sample initial-transition-final stages, and the bond break-no break decision is made via geometrical considerations learned by the machine learning model, with no energy information.

A more general view of a problem addressed by QM/MM schemes is the integration of highly accurate quantum-mechanical calculations with geometrically- and chemically- complex boundary conditions. While a critical region of the chemical reaction or physical process being simulated may be typically enclosed in a high-fidelity simulation region, the behavior of the process may depend on its broader surroundings. The dependence may include: (1) electromagnetic fields generated or screened by the surrounding medium; (2) mechanical stresses or hydrostatic pressure provided by the surrounding medium, (3) geometric constraints imposed by the surrounding environment (e.g., a 2D film, a spherical cavity, or similar), and/or (4) bonding structure ensuring the correct distribution of electrons in a chemical system.

In one or more simulation proposals, the boundary conditions may be approximately provided by one or several of the following approaches: (1) creating a sufficiently large high-fidelity region to minimize the effect of any unphysical effects at the simulation boundaries; (2) adding a lower fidelity region on the external boundary of the high-fidelity region to help increase the system size at a computationally-affordable cost (like the QM/MM approach); (3) imposing periodic boundary conditions to imitate embedding in a near-infinite solid; (4) artificially changing the chemical structure of the simulated material at the boundary to mimic the bonding of a real, much larger system, e.g., adding hydrogen terminations to dangling bonds, and/or (5) adding continuous fields at the system boundary to exert a mean-field effect on the system, thereby mimicking the desired boundary effect, e.g., a dielectric medium or external stress field.

These approaches have drawbacks that make simulating certain systems of interest challenging. Some of the limitations of these approaches include: (1) exceedingly-large computational costs of increasing the size of the high-fidelity QM region; (2) difficulty in ensuring the compatibility of high-fidelity and low-fidelity simulations in the QM/MM approach (e.g., (a) mismatch in energies and forces predicted for equivalent geometries in the two regions, leading to highly unphysical discontinuities at the interface of the two systems; (b) intermixing between the high-fidelity and low-fidelity regions in the case of a liquid or gaseous systems; (c) extreme difficulty in defining a boundary between first and second regions in a solid system to define both the electronic structure of the high-fidelity region and the atomic bonding topology of the low-fidelity region in a physically-meaningful way; and (d) inconsistency in a long-range electrostatic interaction between high-fidelity and low-fidelity regions); (3) periodic-image artifacts created by periodic boundary conditions and requirement that the simulation cell forms a periodic geometry (e.g., a Bravais lattice), which means that the spherical or cylindrical systems are augmented to form a rectangular simulation geometry which has a much larger simulation volume (e.g., in a separate example, amorphous or disordered materials such as glasses and liquids may need extremely large simulation cells to approximate their intrinsically non-periodic structure); (4) lack of general methods to ensure that chemical terminations at the system boundary are physically meaningful, making the simulations very laborious, dependent on expert knowledge, and non-transferrable; and (5) arbitrary nature of the definition of the boundary between an atomistic (e.g., discrete) and continuous description of the system, which creates a problem of an external field (e.g., dielectric medium) fluctuating at the atomistic boundary in a complex and unphysical manner.

Considering the foregoing, one or more machine learning simulation methods (e.g., MLIP learning methods) are needed to address one or more of the drawbacks identified above. One or more of the simulation methods may be used to create computationally controllable, geometrically complex boundary conditions for atomistic simulations for arbitrary systems.

Machine Learning Interatomic Potential Shell Simulation Methods

In one or more embodiments, an atomistic simulation using a machine learning interatomic potential (MLIP) may be performed at high fidelity, with relatively low computational costs and with arbitrary geometrically and chemically complex boundary conditions using a fixed shell simulation setup. The methods of one or more embodiments implement geometrically complex boundary conditions for atomistic simulations by leveraging short range, topology free MLIPs.

A quantitative range for high fidelity computations may vary depending on the property being determined by the calculations and the chemical systems being investigated. In one or more embodiments, the accuracy of a high-fidelity model may be quantified by a mean absolute error (MAE) compared to the training set, which, if training on forces, may be less than 1 meV/Angstrom, which may be an exceptionally good model, or even up to 100 meV/A for an adequate model. In one or more embodiments, the accuracy of a high-fidelity model may be any of the following values or in a range of any two of the following values: 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, and 10%. The computational costs may depend on the size of the chemical system being considered. Relatively low computational cost may be measured in a CPU time of hours to days on a single high-performance core with memory of 10 to 100 GB of RAM and 1 to 100 GB of disk space.

The fixed shell simulation may include one or more of the following components. In one or more embodiments, the first component is a central high-fidelity region. The central high-fidelity region may contain a molecular simulation driven by an MLIP trained to capture one or more chemical and/or physical processes of interest. The molecular dynamics of the MLIP may be driven by one or more methodologies (e.g., Nose-Hoover molecular dynamics, metadynamics, umbrella sampling, etc.), a gradient descent for energy minimization, or any other adjustment of atomic positions.

In one or more embodiments, the second component is a finite-thickness shell of a chemical structure chosen to represent one or more desired chemical boundary conditions. In one or more embodiments, the energetics and/or dynamics of the atoms in the shell may be computed using the same MLIP as the central region. In one or more embodiments, one or both of the following conditions are imposed in the shell relative to the central region: (1) the masses of the atoms are set to infinity, which both fixes the chemical structure of the shell throughout the simulation, and ensures any atomic collisions with the shell are elastic and do not lead to any unphysical energy gain or dissipation from the central simulation region; and (2) the thickness of the shell is set to be equal to or greater than an effective interaction cutoff of the MLIP. Depending on the MLIP used, the effective interaction cutoff may be any of the following values or in a range of any two of the following values: 4, 5, 6, 7, 8, 9, 10, 11, 12, 13, 14, 15, 16, 17, 18 and 19 Å. The thickness of the shell may be any of the following values or in a range of any two of the following values: 4, 5, 6, 7, 8, 9, 10, 11, 12, 13, 14, 15, 16, 17, 18, 19 and 20 Å. In the case of message-passing neural networks, the shell thickness may be a per-layer interaction cutoff multiplied by a number of message-passing layers in the neural network. The shell thickness may be configured to provide a chemically realistic environment for all or some of the atoms in the central high-fidelity region.

In one or more embodiments, the third component is an optional second shell surrounding the first shell of the same or a different chemical structure. In one or more embodiments, the atoms in the second shell are not included in the evaluation of the MLIP and, if present, may serve to define the surroundings of the second shell.

In one or more embodiments, the fourth component is an optional continuous field surrounding the first shell and optionally the second shell if present. The optional continuous field may be configured to provide boundary conditions to any long-range physics included in the MLIP. For instance, a dielectric field may be included to provide a long-range screening for charged systems. The field may also describe a magnetic environment, concentration gradient, chemical environment, or mechanical environment. The field may be discretized to a numerical grid for ease of calculation. The grid size may be anywhere between 0.1 nm to 100 nm.

In one or more embodiments, the first fixed shell is not completely fixed but is allowed to evolve slowly over time. This change allows the shell to respond sluggishly to changes in the continuum region and/or in the other shells, while retaining a reasonable environment for the inner region. For example, the first shell may spontaneously polarize or change density in response to mechanical stress or electric fields from the continuum region and/or from the inner shell. Should it not be practical or possible to directly use the energies and forces obtained from an MLIP for this time evolution because the externally facing atomic environment may terminate in nonphysical ways, the time-evolution of the shell may be implemented using one of the following methods: (1) modulating the lattice length (e.g., simulation cell size) during the simulation; and (2) alternating MLIP steps (e.g., several thousand MLIP steps) with several hundred classical-MD steps to move atoms within the first fixed shell if the first fixed shell changes its atomic positions. With respect to (1), this degree of freedom is compatible with infinite-mass atoms in the first fixed shell and allows small changes in density within the entire box. With respect to (2), the approach may be described using a classical molecular dynamics (MD) simulation. The simulation may include one or more of the following iterations: (1) the classical MD topology may be fixed throughout and the classical MD steps may move atoms in the inner and/or fixed regions if there are no bonds breaking in any region; (2) the classical MD iteration may be defined to move atoms in the fixed region(s) only (e.g., first shell and/or second shell) while assigning an infinite mass to the inner shell atoms if there are no bonds breaking across the boundary between the fixed and inner regions; and (3) a subset of atoms in the fixed region (e.g., water) that respond to electric fields (e.g., polarization) may move during the classical MD steps while keeping the other atoms immobile by assigning them an infinite mass. Iteration (2), defined by temporarily freezing the high-fidelity region and evolving the boundary, allows for a slow response of the boundary to the dynamics of the inner region.

Figure 5 is a schematic view of system geometry 500 of an MLIP simulation according to one or more embodiments. Figure 5 includes a schematic depiction of the boundary conditions imposed on the MLIP driven simulation in central high-fidelity region 502. The thickness of first fixed shell 504 is no less than an interaction range of the MLIP. First fixed shell 504 is configured to provide a chemically-realistic, mechanically-fixed set of boundary conditions for central region 502. Atoms in first fixed shell 504 are included in the MLIP evaluation but are defined to have an infinite mass to ensure elastic collisions at an atomic level. Optional second shell 506 contains a chemically-realistic environment for first fixed shell 504 but is not included in the MLIP evaluation. Optional continuum region 508 satisfies additional long-range boundary conditions (e.g., dielectric screening). In another embodiment, first shell 504, second shell 506, and continuum regions 508 may be defined to vary in time to impose dynamic boundary conditions on central region 502.

In system geometry 500 shown in Figure 5, the following boundary conditions for central high-fidelity region 502 are satisfied: (1) an arbitrary chemical environment, including the termination of dangling bonds, is provided by the inner surface of first fixed shell 504; (2) arbitrary mechanical stress conditions may be provided by changing the mechanical state of first fixed shell 504 exerting hydrostatic, anisotropic, and/or shear stress on central region 502; and (3) arbitrary electric fields and mean-field dielectric conditions may be imposed by controlling the charge state of the fixed shells and optional long-range dielectric medium.

The MLIP simulation of one or more embodiments is agnostic to the specific chemistry of the system in central high-fidelity region 502 and has a computational cost that only scales with the size of central high-fidelity region 502. These beneficial properties are a result of one or more of the following elements of construction of the MLIP.

First, the MLIPs of one or more embodiments have an inherent finite interaction range, such that atoms outside of the fixed interaction range cannot impact each other through the MLIP. By setting the thickness of a fixed shell to be larger than the interaction radius of the MLIP, the simulated atoms (e.g., all the simulated atoms) experience a well-controlled, physically-realistic environment at the system boundary. In one or more embodiments, this property of the MLIP eliminates system-specific terminations to dangling bonds at the outer boundary of the simulation region.

Second, the MLIPs of one or more embodiments define which atoms interact with each other dynamically at each timestep of the simulation. These MLIPs do not have a predefined topology (e.g. bonding network). Therefore, in these embodiments, a physical simulation may proceed when the atomic energy in the vicinity of the atom of interest is realistic. The fixed shell boundary condition of one or more MLIPs of one or more embodiments satisfies this condition for any timestep, without imposing any constraints on the evolution of the central high-fidelity region or introducing any unphysical discontinuities in the system energy and forces near the boundary.

Third, in the MLIPs of one or more embodiments, the properties set forth in the first and second paragraph above, provides a means where only the atoms in the central high-fidelity region and the first fixed shell are included in the numerical evaluation of the MLIP. Therefore, the MLIPs of one or more embodiments are not padded, thereby reducing calculation costs.

In the MLIPs of one or more embodiments, the boundary conditions are fixed in time, so that the only dynamic component of the system is the central high-fidelity region. In an alternative embodiment, time-dependent boundary conditions may be imposed by adding predefined time evolution to the first fixed shell, the second fixed shell, or the external dielectric field. For example, mechanical oscillation may be imposed by a time-dependent compression and expansion of the shells.

**Machine Learning And Molecular Mechanics Simulation Methods**

In one or more embodiments, an electrostatic-embedding machine learning/molecular mechanics (ML/MM) simulation method is disclosed. In the ML/MM simulation method, the local energy and the charge assignment depend on the electrostatic charges in an MM region of an ML/MM scheme. The ML/MM method may be implemented through using the MM charges to generate an effective electric field in an ML region and assigning a charge based on the effective electric field and the atomic environment, thereby enabling long-range interactions in a machine-learned interatomic potential. In one or more embodiments, the charge assignment and the energy prediction training data includes explicit fields.

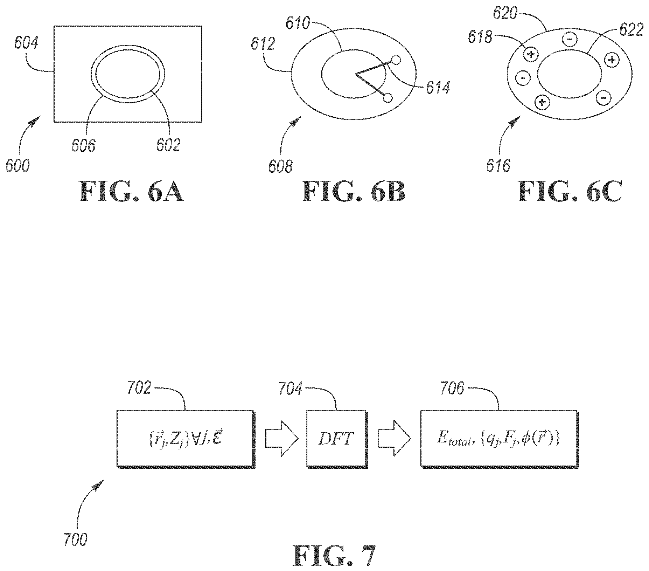

Figure 6A depicts ML/MM method 600 including ML region 602, MM region 604, and interface 606 therebetween. Figure 6B depicts mechanical embedding method 608 where the ML solver does not account for charge outside of ML region 610 in interface 612 but bonds 614 are visible to the ML solver. Figure 6C depicts electrostatic embedding method 616 where some charges 618 in interface 620 are visible outside ML region 622 to repolarize atoms.

The electrostatic-embedding ML/MM simulation method may include one or more of the following steps. These steps may be rearranged, modified, and/or omitted based on the implementation of the electrostatic-embedding ML/MM simulation method.

In one or more embodiments, the first step of the electrostatic embedding ML/MM simulation method includes generating training data. Figure 7 depicts information path 700 for a ML/MM simulation method. Information path 700 includes inputs 702, electronic calculations 704, and outputs 706. As depicted in Figure 7, inputs 702 include a set of atoms j with positions $r_j$ and atomic species $Z_i$ in an electric field E of direction k. Inputs 702 are input into a DFT calculation, outputting a total energy $E_{total}$ and forces on each atom and charges on each atom according to a charge scheme (e.g., a Hirshfeld, iterative Hirshfeld, or ChElPG charge scheme). Outputs 706 may also include an electrostatic potential $\phi$ at each point r of the region.

In a first sub-step, the electrostatic forces and energy may be subtracted before training to provide that the ML model captures only the non-electrostatic effects, and all electrostatics are included in analytical form. The electrostatic forces and energy may be given from $q_j$ that are computed with a DFT or similar electronic calculation.

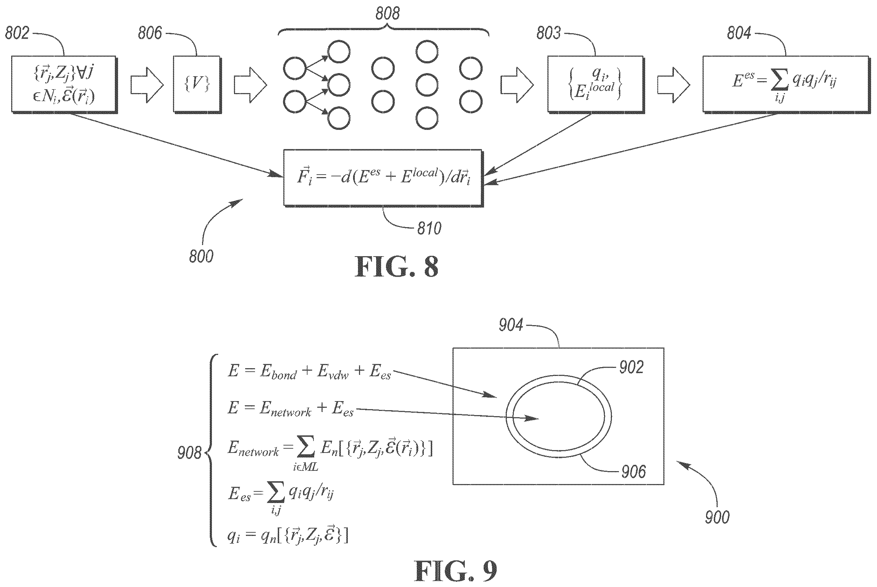

In a second step according to one or more embodiments, the training data generated from step one is used to a train an MLIP. Figure 8 depicts training method 800 for training an MLIP according to one embodiment. Input 802 at each step of training method 800 includes the set of atoms j in a neighborhood of an atom i including their positions $r_i$ and species $Z_j$ and an applied electric field at that position given by equation (6) below.

$$\vec{\mathcal{E}}(\vec{r}_i) = \frac{1}{\epsilon} \nabla_r \sum_{j \in MM} q_j / r_{ij} \qquad (6)$$

In one or more embodiments, each snapshot for the training data has the same electric field for all atoms. The permittivity $\epsilon$ may be declared or learned. The far-field charges may generate a local electric field as depicted in block 803 used to predict the charges and/or energy. Output 804 of training method 800 is the total energy, the force on each atom $j$, the assigned charge $q_j$, and optionally the electrostatic potential. Training method 800 also includes embedding 806 and learning 808 within a local and/or charge network. Training method 800 also includes backpropagation 810 where input 802, block 803, and output 804 backpropagate. In one or more embodiments, the MLIP may be first and second MLIP networks separate from each other. In other embodiments, the MLIP may be a single network with multiple heads. In some embodiments, the MLIP is a Gaussian process.

In one or more embodiments, step three is a production step. The production step may define a ML region and an MM region spatially distinct from each other. In the MM region, bonds may be assigned between atoms and charges may be constant. The bonded energy is typically a combination of bond lengths, bond angles, and torsion/dihedral terms between bonded atoms. The unbonded energy is typically an electrostatic contribution from the charges plus a van der Waals interaction in the short range. The charges in the MM region may be combined to form an electrostatic potential and electric field for the ML region. Each atom i in the ML region may be labeled with an appropriate electric field according to equation (6). The ML network may take electrostatic information from the MM region, as well as the atomic positions and species from the ML and interface regions. The ML network may output the energies, forces, and/or electrostatic charge assignments for the atoms in the ML region so that every atom has a charge assignment. The total energy may be comprised of an MM bonded and van der Waals interactions, the ML short-range interaction, and the electrostatic interactions of all atoms.

Figure 9 depicts ML/MM simulation method 900 including ML region 902, MM region 904, and interface 906 therebetween where ML/MM simulation method 900 may be used to calculate total energy E from equations 908 shown in Figure 9. Total energy E may be used in one or more molecular dynamics (MD) steps (e.g., leapfrog update for NVE (number of particles, volume, and energy) integration, transition state search using a saddle point calculator; Monte Carlo step using total energy, etc.).

**Double Tier Machine Learning In-Space Hybrid Simulation Methods**

In one or more embodiments, a double tier machine learning/machine learning (ML/ML) hybrid simulation method is disclosed that addresses one or more of the shortcomings identified herein relating to proposed machine learning methods (e.g., MLIP). An MLIP may be highly accurate with respect to ground truth calculations, yet at least two orders of magnitudes slower than classical force fields or may consist of less accurate but faster machine learning (ML) atomistic models. The methods of one or more embodiments efficiently simulate atoms in a material system using a combination of machine learning models, where the different models provide varying levels of physical accuracy and computational costs. In one or more embodiments, the application of the two or more models throughout the simulation, as well as the interpolation between the models, may be defined spatially around a region of particular interest, e.g., with a central high-accuracy region smoothly transitioning to an extended, lower-accuracy surrounding environment.

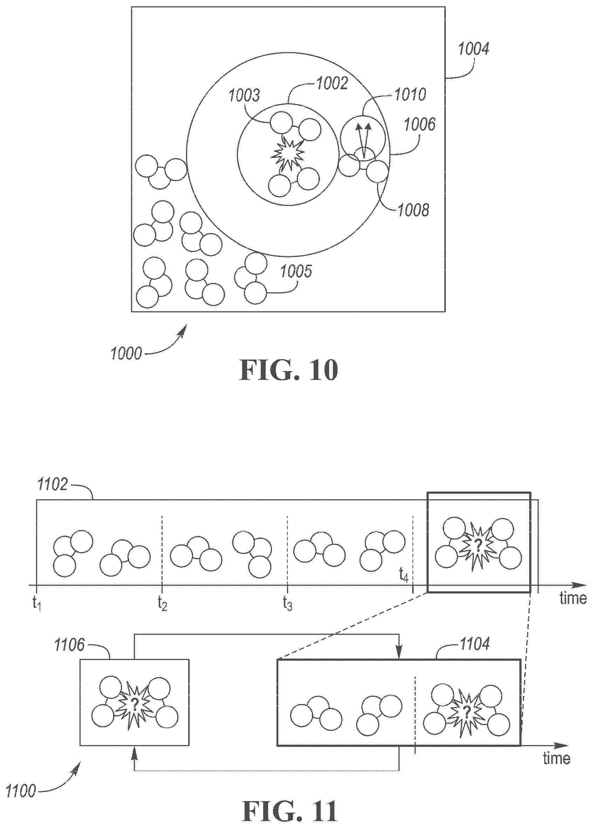

Figure 10 depicts a schematic diagram of in-space hybrid model 1000. In-space hybrid model 1000 may carry out highly accurate computations of atomistic quantities of interest (e.g., energies and forces) within an assigned spatial volume while retaining a faster inference time within a remaining volume, thereby decreasing overall total computational time. While Figure 10 depicts spherical shells for the atomic geometries, these are not limiting geometries and other geometries may used such as slabs or parallelepiped.

A relatively high-cost high-fidelity model, which may be referred to as MLM1, may be employed to predict atomistic quantities of interest (e.g., energies and/or forces) to a high degree of accuracy. The MLM1 may possess a high level of accuracy and a strict local-in-space cutoff such as Allegro. As shown in Figure 10, first region 1002 includes the high-cost, high-fidelity ML model, MLM1. First region 1002 includes atomic geometries 1003.

A lower-cost, lower-fidelity model, which may be referred to as MLM2, may be employed in a remaining volume (e.g., second region 1004 as shown in Figure 10) to provide an appropriate atomistically-resolved environment (e.g., not a mean field environment) to first region 1002. A non-limiting example of a low interest spatial volume where high fidelity is not required is a general low-density medium. Second region 1004 include atomic geometries 1005.

As the MLM2 is less expensive to compute than the MLM1, the MLM2 may be applied to the remaining volume (e.g., second region 1004), but it may also be applied to the entirety of the volume (e.g., first and second regions 1002 and 1004), thereby increasing overlap region 1006 between first and second regions 1002 and 1004. Overlap region 1006 may be used to generate MLM1 and MLM2 double predictions. The MLM2 may possess a relatively high inference speed and a strict local-in-space cutoff. The MLM2 may be implemented using FLARE.

The MLM2 may not be restricted to a kernel-based model such as FLARE but it may also be the same framework as the MLM1, but with less "strict" hyperparameters thereby producing faster inference speeds. As a non-kernel-based example, the MLM1 may use an Allegro framework with an angular resolution L=3, while the MLM2 has an angular resolution of L=0 or L=1. The MLM2 may be a mapped (e.g., spline) model. In one or more embodiments, both MLM1 and MLM2 frameworks possess a strict spatial locality. In one or more embodiments, the locality of the models produces the absence of long-range effects during inference and/or the freedom to carve out atomic neighbors of interest freely without far-field effects.

In one or more embodiments, MLM1 and MLM2 are trained on the same training data. In another embodiment, MLM2 may be trained in a subset of training set of MLM1. Using this training method, the MLM2 framework may be implemented using a kernel-based method, thereby limiting the training dataset size relative to a non-kernel-based method. In both instances, the total or partial overlap of the training data may provide one or more benefits. For example, the total or partial overlap of the training data reduces or eliminates large or systematic differences in geometry prediction between the high-fidelity and low-fidelity regions (e.g., lattice mismatches). Another benefit in one or more embodiments is the reduction in computational costs because additional training data is not required.

In one or more embodiments, the hybrid ML-ML model has a boundary volume (e.g. overlap region 1006) where both MLM1 and MLM2 are evaluated on atomic geometries (e.g., atomic geometries 1008) that are present within that volume. An example size of the boundary volume is the closest atom within the exclusive MLM1 region within its full cutoff can see only boundary atoms (e.g., atoms within the boundary volume).

First and second predictions at the boundary volume may be used to construct an error function. If MLM1 and MLM2 are trained on the same training datasets, the atomistic quantity of interest (e.g., forces) may be nearly identical. The difference in prediction (e.g., forces 1010 of MLM1 and MLM2 mismatching) via a user-defined hyperparameter may enable an active learning procedure by setting a user defined error threshold. If the error function crosses a certain threshold, a carve-out of the volume around the high-uncertainty atoms may be selected and computed with a higher accuracy method (e.g., DFT) with the labeled data added to the training set of MLM1 and/or MLM2. In one embodiment, MLM1 and MLM2 are immediately re-trained. In another embodiment, the MLM1 and MLM2 models continue to sample the phase space until a threshold number of DFT calls have been made and only selectively re-train for computational efficiency.

An example of an error function may be a normalized aggregate dot product between the forces (with ceiling N, where N is the number of atoms), or per atom dot product between forces, equal to 1 if all per-atom force predictions are aligned. If the difference in prediction between MLM1 and MLM2 is within a user defined bound, the difference between MLM1 and MLM2 may be adjusted (e.g., smeared) such that at the surface of the boundary volume in contact with the MLM1 region, the prediction is the MLM1 prediction, while the opposite prediction at the other boundary.

The interpolation between the pure MLM1/overlap surface and the overlap/pure MLM2 surface may be performed at inference to ensure continuities. The interpolation may be performed using a linear weight according to equation (7) set forth below.

$$F_i = x_i F_1 + (1 - x_i) F_2 \qquad (7)$$

where x is a fractional depth of an atom i toward the MLM1 region. In another embodiment, the interpolation may be performed nonlinearly according to equation (8) set forth below.

$$F_i = g(x_i) F_1 + \big(1 - g(x_i)\big) F_2 \qquad (8)$$

where g(x) is a possibly nonlinear functions with conditions according to the following equations (9).

$$g(0) = 0, g(1) = 1, g(x) = 1 - g(1 - x) \qquad (9)$$

where it is symmetric across x=1/2.

In-Time Machine Learning Hybrid Simulation Methods

In one or more embodiments, an in-time machine learning hybrid simulation model is disclosed. The in-time machine learning hybrid model may overcome a non-reactive limit of many fast but nonreactive atomistic interatomic potentials by monitoring a flagging variable. If the flagging variable is positive, then a machine learning model capable of assessing the reaction progress is called, or in turn, calling a highest accuracy but lowest efficiency fall-back method to sample the energetics and/or transition state information.

In one or more embodiments, the simulation method may efficiently simulate atoms in a chemical system that may occasionally undergo reactive bond formation or breakage, changing their bonding topology. In one or more embodiments, a hierarchy of methods at increasing computational cost and accuracy are utilized to realize the simulation method. The evolution of simulation may be switched between timesteps among (1) an efficient, low-accuracy classical molecular dynamics approach such as a CHARMM bonded force field, (2) a machine learning interatomic potential such as a FLARE Gaussian Process, and (3) an electronic structure method such as density-functional theory. The switch may be implemented based on whether the system is likely to be undergoing a reaction, and/or whether this reaction has been previously calculated and incorporated into the machine-learning model training.

Figure 11 depicts a schematic diagram of in-time hybrid model 1100. Block 1102 depicts a fast but low fidelity model having non-reactive interatomic potentials to generally evolve positions of atoms at different times ($t_1$, $t_2$, $t_3$, and $t_4$). This model may use a classical molecular dynamics (CMD) approach. When a flagging event is detected (e.g., at $t_4$), block 1104 is called. Block 1104 depicts a slower but higher fidelity machine learning model (MLM). The model depicted by block 1104 is slower than the model depicted by block 1102 but higher fidelity than the model depicted by block 1102. Block 1104 is configured to sample the trajectory in temporal proximity to the flagged event.

In one or more embodiments, there are two possible outputs from block 1104. If the geometries at play have been sampled before by block 1104, a react versus no-react output may be made based on the energetics of the geometry, and the bond formation (or lack thereof) is transferred to the topology used by block 1102. Conversely, if block 1104 is uncertain about the trajectory, a slowest but highest fidelity model (e.g., an electronic structural model (ESM)) (block 1106) is called to iteratively train the model of block 1104. In one or more embodiments, the model of block 1106 is slower than the models of blocks 1102 and 1104 but higher fidelity than the models of blocks 1102 and 1104.

In another embodiment, an in-time hybrid model may be biased (e.g., pushed) towards one or more bonding events. For instance, the in-time hybrid model may be configured to mimic a polymerization reaction to produce a reaction product (e.g., a polymer chain or a cross-linked network).

A flagging mechanism may be any on-the-fly computed variable configured to trigger a bonding reaction. Non-limiting examples of such variable may include bond lengths, bond distortions, potential energy, kinetic energy, and/or forces, a surrogate kernel-based model with principled uncertainty (e.g., Flare), or a smooth overlap of atomic positions (SOAP) descriptor vector.

The slower but higher fidelity model of block 1104 may be strictly local (e.g., Allegro or FLARE) to provide the absence of long-term effects during inference and/or provide the freedom to carve out atomic neighbors of interest without far-field effects. In one or more embodiments, the size of the carve out for the slower but higher fidelity model of block 1104 may be two to five times (e.g., two, three, four, or five times) the hard cutoff of the slower but higher fidelity model of block 1104 from the center of the flagging atom. The exact amount may be an input parameter chosen on the basis that at least within a full cutoff of the atom, bond, or pair that triggered the flagging mechanism. The atoms and their neighbors may be included for an accurate representation of the atomic configuration.

In one or more embodiments, during the simulation of the model of block 1104, the model is required to have access to some uncertainty quantification so that it can determine whether that specific geometry-to-reaction has been explored already or not. If a kernel-based method (e.g., FLARE) is used for the model of block 1104, the intrinsic uncertainty may be used. If a neural network-based method (e.g., Allegro) is used for the model of block 1104, either the uncertainty among 2 to 6 models (e.g., 2, 3, 4, 5, or 6 models) may be used (e.g., an ensemble uncertainty for neural networks), or Gaussian mixture models trained on a network's learned features may be used. If the model of block 1104 determines it knows a reaction pathway well enough, it may determine whether a bond is added or not based on known energetics. For example, this decision may be made by Boltzmann weighting with the energy barrier between states. The weighting may be biased to force the reactions to happen such as in the case where we may want to drive the system towards reacting.

Conversely, if the model of block 1104 determines it is too "uncertain" based on previous sampled geometries, the model of block 1106 may be called. In one or more embodiments, this route triggers an active learning loop so that the model of block 1104 may be fully re-trained to include new geometry-to reaction information. In the case that the geometry-to-reaction is known, a counter for that specific pathway may be increased. Otherwise, a new pathway may be added with the counter being reset to 1. This process may be useful to map the zoology of possible reactions (e.g., a mapping of reactants and products including energies, reactants, products, pathways, and/or barriers), together with its ESM calculations supporting it from which reaction barriers may be extracted via calculations (e.g., nudged elastic bond (NEB) calculations). The NEB calculations may be performed as part of an active loop between the models of block 1104 (e.g., MLM) and block 1106 (e.g., ESM) and added to an expanded training set to retrain the model of block 1104 (e.g., MLM). Committor-type approaches may be employed to sample one or more rare transition states. The committor-type approach may be used to study rare events and transitions between metastable states in complex systems. Once a saddle point (e.g., a transition point) is determined, sampling may be conducted using an MLIP or ESM to assemble a transition rate and to add to the zoology.

The model of block 1102 may be restarted once a decision is made to make a bond or not make a bond. In one or more embodiments, the model of block 1102 may be restarted at $t_3$ shown in Figure 11. In another embodiment, the model of block 1102 may restarted from the bonded or not-bonded version of $t_4$ (e.g., the $t_4$ snapshot where the bond has been added explicitly). In this alternative embodiment, a temporal smearing of the interaction may be imposed on the new topology to provide simulation stability and/or geometry continuity. Once a bond function (e.g., a harmonic bond in a CMD simulation) is added between two atoms not bonded prior to the call to the model of block 1104, such interaction may be modulated over time so that over the course of a number of timesteps (e.g., on the order of 100s) the strength of the new interaction goes from not existing (e.g., 0) to fully bonded (e.g., 1). In one embodiment, the transition in the new interaction strength may be achieved by slowly ramping up the value of the spring constant applied to the above example of harmonic bonding. In the opposite case (i.e., no bond is made), the model of block 1102 can proceed without further adjustment.

The model of block 1102 may be configured to obtain final structures resulting from reaction chains (e.g., cross-linking of a polymer). The model of block 1102 may be configured to automatically and efficiently sample possible reaction pathways given an initial atomic geometry.

As shown in Figure 11, the models of block 1104 and blocks 1106 create a loop. The loop may be used to provide reaction energetic considerations for the model of block 1102. The loop may also be used to sample and bucket the reactions to generate a database of possible reactions. The database of possible reactions may include relative initial, final, and transition states, and reaction energy barriers.

In one or more embodiments, the model of block 1102 is a CMD and the model of block 1104 is an MLIP. The transition between the CMD and the MLIP may be performed continuously using a weighting factor over some time t. For example, for forces F, the weighting factor may be represented by the following equation (10):

$$F = F_{MLIP}\, g(t/\tau) + F_{CMD}\big(1 - g(t/\tau)\big) \qquad\qquad (10)$$

where g(x) is a linear or nonlinear function with the boundary conditions $g(0)=0$ and $g(1)=1$ when entering the MLIP domain, and similar when exiting the MLIP domain. $\tau$ is a characteristic timescale that identifies the time at which the MLIP is fully active.

The output of the machine learning simulation methods of one or more embodiments may be used as actuation signals for controlling one or more chemical systems. The actuation signals may be transmitted to control the one or more chemical systems. Non-limiting examples of chemical systems include those relating to ionic transport, chemical reactions, and material bulk and surface degradation in material systems, such as, devices or functional materials. The chemical systems may be material systems such as fuel cells, surface coatings, batteries, water desalination, and water filtration.

The machine learning simulation methods of one or more embodiments may be used to derive or predict one or more physical states of interaction (e.g., dynamic motion of the atoms) between existing real objects (e.g., atoms) from measurement of physical properties (e.g., a set of atomic positions and atomic species, including atomic number and/or isotope). The machine learning simulation methods of one or more embodiments may be used to analyze interactions within or between materials.

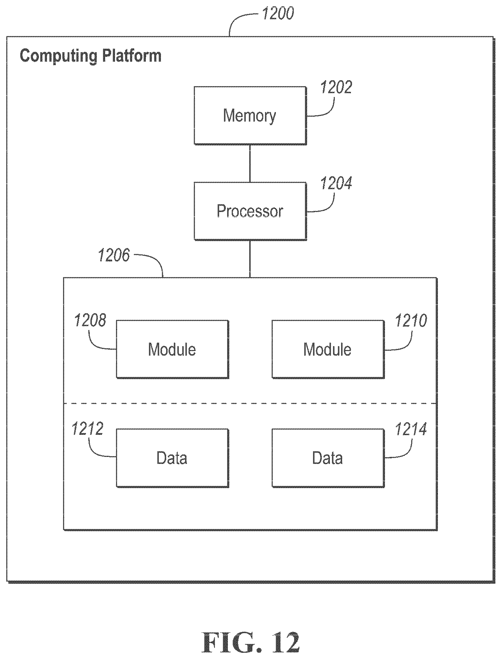

The machine learning simulation methods of one or more embodiments are implemented using a computing platform, such as the computing platform 1200 as illustrated in Figure 12. The machine learning simulation methods may be implemented as part of a computational software suite. The computing platform 1200 may include memory 1202, processor 1204, and non-volatile storage 1206. The processor 1204 may include one or more devices selected from high-performance computing (HPC) facilities including high-performance cores, microprocessors, micro-controllers, digital signal processors, microcomputers, central processing units, field programmable gate arrays, programmable logic devices, state machines, logic circuits, analog circuits, digital circuits, or any other devices that manipulate signals (analog or digital) based on computer-executable instructions residing in memory 1202. A HPC facility may include advanced computing hardware and software configured to perform computationally intensive tasks at much higher speeds than a typical desktop or server computer. The HPC facility may deliver up to one (1) million random read input/output operations per second (IOPS).

Memory 1202 may include a single memory device or a number of memory devices including, but not limited to, random access memory (RAM), volatile memory, non-volatile memory, static random access memory (SRAM), dynamic random access memory (DRAM), flash memory, cache memory, or any other device capable of storing information. The non-volatile storage 1206 may include one or more persistent data storage devices such as a hard drive, optical drive, tape drive, non-volatile solid-state device, cloud storage or any other device capable of persistently storing information.

Processor 1204 may be configured to read into memory 1202 and execute computer-executable instructions residing in software modules 1208 and 1210 of the non-volatile storage 1206 and embodying iterative machine learning methods of one or more embodiments. Software modules 1208 and 1210 may include operating systems and applications. Software modules 1208 and 1210 may be compiled or interpreted from computer programs created using a variety of programming languages and/or technologies, including, without limitation, and either alone or in combination, Java, C, C++, C#, Objective C, Fortran, Pascal, Java Script, Python, Perl, and PL/SQL.

Upon execution by the processor 1204, the computer-executable instructions of software modules 1208 and 1210 may cause the computing platform 1200 to implement one or more of the iterative machine learning methods of one or more embodiments disclosed herein. The non-volatile storage 1206 may also include data 1212 and 1214 supporting the functions, features, and processes of the one or more embodiments described herein.

The program code embodying the machine learning simulation methods described herein is capable of being individually or collectively distributed as a program product in a variety of different forms. The program code may be distributed using a computer readable storage medium having computer readable program instructions thereon for causing a processor to carry out aspects of one or more embodiments. Computer readable storage media, which is inherently non-transitory, may include volatile and non-volatile, and removable and non-removable tangible media implemented in any method or technology for storage of information, such as computer-readable instructions, data structures, program modules, or other data. Computer readable storage media may further include RAM, ROM, erasable programmable read-only memory (EPROM), electrically erasable programmable read-only memory (EEPROM), flash memory or other solid state memory technology, portable compact disc read-only memory (CD-ROM), or other optical storage, magnetic cassettes, magnetic tape, magnetic disk storage or other magnetic storage devices, or any other medium that can be used to store the desired information and which can be read by a computer. Computer readable program instructions may be downloaded to a computer, another type of programmable data processing apparatus, or another device from a computer readable storage medium or to an external computer or external storage device via a network.

Computer readable program instructions stored in a computer readable medium may be used to direct a computer, other types of programmable data processing apparatus, or other devices to function in a particular manner, such that the instructions stored in the computer readable medium produce an article of manufacture including instructions that implement the functions, acts, and/or operations specified in the flowcharts or diagrams. In certain alternative embodiments, the functions, acts, and/or operations specified in the flowcharts and diagrams may be re-ordered, processed serially, and/or processed concurrently consistent with one or more embodiments. Moreover, any of the flowcharts and/or diagrams may include more or fewer nodes or blocks than those illustrated consistent with one or more embodiments.

The following application is related to the present application: U.S. Pat. Appl. Ser. No. (RBPA0522PUS) filed on _, U.S. Pat. Appl. Ser. No. (RBPA0514PUS) filed on _, and U.S. Pat. Appl. Ser. No. _ (RBPA0520PUS) filed on _, which are incorporated by reference in their entirety herein.

While all of the invention has been illustrated by a description of various embodiments and while these embodiments have been described in considerable detail, it is not the intention of the applicant to restrict or in any way limit the scope of the appended claims to such detail. Additional advantages and modifications will readily appear to those skilled in the art. The invention in its broader aspects is therefore not limited to the specific details, representative apparatus and method, and illustrative examples shown and described. Accordingly, departures may be made from such details without departing from the spirit or scope of the general inventive concept.

**Claims**

1.   A machine learning interatomic potential (MLIP) method of determining a physical state of interaction between atoms from one or more physical properties of the atoms, the method comprising:
assigning a charge $q_i$ to a first subset of atoms via the MLIP dependent on a nonconstant field generated by a second subset of atoms having a charge $q_j$ to determine the physical state of interaction between the atoms.

2.   The MLIP method of claim 1, wherein the assigning step is carried out using a neural network.

3.   The MLIP method of claim 1, wherein the assigning step is carried out using a Gaussian process.

4.   The MLIP method of claim 1, wherein the assigning step includes assigning the charges $q_i$ and $q_j$, an energy $E_i$ for the first subset of atoms, and an energy $E_j$ for the second subset of atoms.

5.   The MLIP method of claim 4, wherein the assigning step is carried out as two or one head of a neural network.

6.   The MLIP method of claim 1, wherein the physical state of interaction between the atoms is selected from the group consisting of energy, force, stress, pressure, electric field, magnetic field, chemical potential, and combination thereof.

7.   The MLIP method of claim 1, wherein the assigning step is carried out in a molecular dynamics simulation of the atoms.

8.   The MLIP method of claim 1, wherein the assigning step includes defining a machine learning region and a molecular mechanics region spatially distinct from the machine learning region.

9.   The MLIP method of claim 1, wherein the assigning step is carried out in a Monte Carlo (MC) simulation of the atoms.

10.  The MLIP method of claim 1, wherein the physical state of interaction is minimized energy of the atoms.

11.  The MLIP method of claim 1, wherein the first subset of atoms undergoes a chemical reaction.

12.  The MLIP method of claim 1, wherein the first subset of atoms undergoes a polarization.

13.  The MLIP method of claim 1 further comprising simulating the second subset of atoms using an analytical energy function.

14.  The MLIP method of claim 1 further comprising simulating the second subset of atoms using a bonded topology.

15.  The MLIP method of claim 1 further comprising simulating the second subset of atoms using fixed charges.

16.  The MLIP method of claim 1 further comprising receiving training data for the MLIP.

17.  The MLIP method of claim 16 further comprising training the MLIP using the training data.

18. The MLIP method of claim 17, wherein the training data includes a number of snapshots including the same electric field for all of the atoms.

19. A machine learning interatomic potential (MLIP) method of determining a physical state of interaction between atoms from one or more physical properties of the atoms for use in a chemical system, the method comprising:

assigning a charge $q_i$ to a first subset of atoms via the MLIP dependent on a nonconstant field generated by a second subset of atoms having a charge $q_j$ to determine the physical state of interaction between the atoms; and using the physical state of interaction between the atoms to control the chemical system.

20. A machine learning interatomic potential (MLIP) method of determining a physical state of interaction between atoms from one or more physical properties of the atoms, the method comprising:
assigning charges to a first subset of atoms in a molecular mechanics region via the MLIP to generate an electric field in a machine learning region to determine the physical state of interaction between the atoms.

100

102 106 108 104

$$\{\vec{r}_j, Z_j\} \Rightarrow \{V\} \Rightarrow \boxed{\text{NETWORK}} \Rightarrow E$$

## FIG. 1

200

204 208 210 206

$$\{\vec{r}_j, Z_j\} \Rightarrow \{V\} \Rightarrow$$

$$\vec{F}_i = -dE / d\vec{r}_i$$

## FIG. 2

302 Atomic Positions $\{\vec{r}\}$

Update Partitioning? → Partition Into Processors 304

Update Neighbor List? → Create Neighbor Lists $\{\vec{r}_j\}$ 306

Compute Energy
$E(\{\vec{r}_j, Z_j\})$ and Forces
$\vec{F}_i(\{\vec{r}_j, Z_j\})$ 308

Integration, e.g. Leapfrog

MPI
Communication

Update Positions $\{\vec{r}_j\}$ 310

300

## FIG. 3

EP 4 704 093 A1

FIG. 4A

FIG. 4B

FIG. 4C

FIG. 5

604

600

606    602

**FIG. 6A**

610

612

614

608

**FIG. 6B**

620

618    622

616

**FIG. 6C**

702    704    706

$\{\vec{r}_j, Z_j\} \forall j, \vec{\mathcal{E}}$    $DFT$    $E_{total}, \{q_j, F_j, \phi(\vec{r})\}$

700

**FIG. 7**

808

802
$$\{\vec{r}_j, Z_j\} \forall j \in N_i, \vec{\mathcal{E}}(\vec{r}_i)$$

806
$$\{V\}$$

803
$$\begin{Bmatrix} q_i, \\ E_i^{local} \end{Bmatrix}$$

804
$$E^{es} = \sum_{i,j} q_i q_j / r_{ij}$$

810
$$\vec{F}_i = -d(E^{es} + E^{local})/d\vec{r}_i$$

800

**FIG. 8**

904

902

906

900

908
$$E = E_{bond} + E_{vdw} + E_{es}$$

$$E = E_{network} + E_{es}$$

$$E_{network} = \sum_{i \in ML} E_n[\{\vec{r}_j, Z_j, \vec{\mathcal{E}}(\vec{r}_i)\}]$$

$$E_{es} = \sum_{i,j} q_i q_j / r_{ij}$$

$$q_i = q_n[\{\vec{r}_j, Z_j, \vec{\mathcal{E}}\}]$$

**FIG. 9**

**FIG. 10**

**FIG. 11**

FIG. 12

Europäisches
Patentamt

European
Patent Office

Office européen
des brevets

# EUROPEAN SEARCH REPORT

**Application Number**

EP 25 19 9108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZINOVJEV KIRILL ET AL: "emle-engine : A Flexible Electrostatic Machine Learning Embedding Package for Multiscale Molecular Dynamics Simulations", JOURNAL OF CHEMICAL THEORY AND COMPUTATION: JCTC, vol. 20, no. 11, 28 May 2024 (2024-05-28), pages 4514-4522, XP093346820, US ISSN: 1549-9618, DOI: 10.1021/acs.jctc.4c00248 Retrieved from the Internet: URL:https://pubs.acs.org/doi/pdf/10.1021/acs.jctc.4c00248> | 1-17,19, 20 | INV. G16C10/00 G16C20/30 G16C20/70 |
| A | * p. 4514-4516, p. 4520 * & Zinovjev Kirill: "Electrostatic Embedding of Machine Learning Potentials", Journal of chemical theory and computation: JCTC, vol. 19, no. 6, 23 February 2023 (2023-02-23), pages 1888-1897, XP093346818, US ISSN: 1549-9618, DOI: 10.1021/acs.jctc.2c00914 Retrieved from the Internet: URL:https://pubs.acs.org/doi/pdf/10.1021/acs.jctc.2c00914> * p. 1891, right column, par. 3 * ----- -/-- | 18 | |
| | | | **TECHNICAL FIELDS SEARCHED (IPC)** G16C |

The present search report has been drawn up for all claims

| Place of search | Date of completion of the search | Examiner |
|---|---|---|
| Munich | 2 January 2026 | Mühlbauer, Max |

EPO FORM 1503 03.82 (P04C01)

| Europäisches Patentamt European Patent Office Office européen des brevets | **EUROPEAN SEARCH REPORT** | **Application Number** EP 25 19 9108 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BÖSELT LENNARD ET AL: "Machine Learning in QM/MM Molecular Dynamics Simulations of Condensed-Phase Systems", JOURNAL OF CHEMICAL THEORY AND COMPUTATION: JCTC, vol. 17, no. 5, 5 April 2021 (2021-04-05), pages 2641-2658, XP093349542, US ISSN: 1549-9618, DOI: 10.1021/acs.jctc.0c01112 Retrieved from the Internet: URL:https://pubs.acs.org/doi/pdf/10.1021/acs.jctc.0c01112> * p. 2641 p. 2643 p. 2646 p. 2652 * | 1-20 | |
| A | ANSTINE DYLAN M. ET AL: "Machine Learning Interatomic Potentials and Long-Range Physics", THE JOURNAL OF PHYSICAL CHEMISTRY A, vol. 127, no. 11, 21 February 2023 (2023-02-21), pages 2417-2431, XP093346792, US ISSN: 1089-5639, DOI: 10.1021/acs.jpca.2c06778 Retrieved from the Internet: URL:https://pubs.acs.org/doi/pdf/10.1021/acs.jpca.2c06778> * the whole document, in particular p. 2427 * | 1-20 | TECHNICAL FIELDS SEARCHED (IPC) |

The present search report has been drawn up for all claims

| Place of search | Date of completion of the search | Examiner |
|---|---|---|
| Munich | 2 January 2026 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)